Europäisches Patentamt

European Patent Office

Office européen des brevets

(11) Publication number: **0 317 006 B1**

# EUROPEAN PATENT SPECIFICATION

(45) Date of publication of patent specification: **16.06.93**  (51) Int. Cl.5: **C01B 33/34**, B01J 21/16, B01J 32/00, C07C 5/22

(21) Application number: **88202528.1**

(22) Date of filing: **10.11.88**

(54) **Process for the manufacture of synthetic saponites.**

(30) Priority: **13.11.87 GB 8726661**

(43) Date of publication of application:
**24.05.89 Bulletin 89/21**

(45) Publication of the grant of the patent:
**16.06.93 Bulletin 93/24**

(84) Designated Contracting States:
**BE CH DE ES FR GB IT LI NL SE**

(56) References cited:

J. CHEM. SOC. (A), 1970, pages 1531-1537,
Letchworth, GB; R.M. BARRER et al.:
"Chemistry of soil minerals. Part VIII. Syn-
thesis and properties of fluorhectorites"

BULL. SOC. FR. MINERAL. CRISTALLOGR.,
vol. 93, 1970, pages 449-469, Paris, FR; D.
BORDEAUX-JUGLARET et al.: "Synthèse
sèche d'une smectite fluoréc saturée au so-
dium"

(73) Proprietor: SHELL INTERNATIONALE RE-
SEARCH MAATSCHAPPIJ B.V.
Carel van Bylandtlaan 30
NL-2596 HR Den Haag(NL)

(72) Inventor: **Breukelaar, Johan**
**Badhuisweg 3**
**NL-1031 CM Amsterdam(NL)**
Inventor: **Kellendonk, Frans Joris Antonius**
**Badhuisweg 3**
**NL-1031 CM Amsterdam(NL)**
Inventor: **Van Santen, Rutger Anthony**
**Badhuisweg 3**
**NL-1031 CM Amsterdam(NL)**

Rank Xerox (UK) Business Services
(3.10/3.6/3.3.1)

**Description**

The present invention relates to the manufacture of synthetic saponites, to saponites thus prepared and to their use as carriers and/or catalysts in (hydro)conversion processes such as hydroprocessing, in particular hydrocracking, (hydro)isomerisation, oligomerisation processes and catalytic cracking.

There is considerable interest in so-called swellable clays which can be used, inter alia, in the preparation of pillared clays which are considered promising alternatives to zeolites, in particular since larger pores can be obtained than available with zeolites, provided a proper choice of pillaring materials has been made.

Saponites belong to the family of smectic clays, i.e. swellable compounds, which also include montmorillonites, hectorites, nontronites and beidellites. Both fluorine-containing and fluorine-free saponites are known.

An interesting structural feature concerning saponites, normally represented by the formula

$$A_{x/n}^{n+}[(Mg_3)(Si_{4-x}Al_x)O_{10}Z_2] \cdot mH_2O \qquad (I)$$

wherein A usually represents lithium, sodium or calcium, x represents a value between 0.05 and 0.95, n represents the valency of A, Z represents a fluorine or a hydroxyl group and m has typically a value between 2 and 5, is that magnesium is situated in the octahedral layer at the centre of the triple layer sheet of the smectic clay.

The saponites are also structurally related to the hectorites which do not contain aluminium and wherein besides magnesium, lithium is also present in the octahedral layers.

It is known from R.M. Barrer and D.L. Jones (J. Chem. Soc. A, 1970, pages 1531-1537) to produce predominantly Li-hectorites using solid state synthesis at 800 °C. It was reported therein that at temperatures of about 1000 °C the less interesting alkali amphiboles are predominantly produced and that when reactions are carried out just above the melting conditions large excesses of lithium fluoride have to be used in order to produce a Li-hectorite. The use of sodium fluoride apparently does not lead to the formation of swellable products. The preparation of hectorites at very high temperatures of at least 1150 °C is reported in Bull. Soc. for Mineral Crystallography (1970), 93, pages 449-469. However, stringent conditions, including a neutral or reducing environment and rapid cooling are necessary to produce such materials.

It has now surprisingly been found that synthetic saponites and derivatives thereof can be prepared in high yields and with high crystallinity when use is made of certain alkali halides provided that a solid state synthesis is carried out at a temperature between the lowest melting temperature of the alkali halide(s) concerned and not more than 200 °C below said temperature.

The present invention therefore relates to a process for the manufacture of synthetic saponites according to the general formula:

$$A_{(x+z)/n}^{n+}[(Mg_{3-y}M_y)(Si_{4-x}Al_x)O_{10}Z_2] \qquad (II)$$

wherein A represents any metal ion having basic or amphoteric properties, M represents a bivalent metal ion having an ionic radius between 0.050 nm and 0.085 nm or lithium, Z represents a fluoride moiety, n represents the valency of A, x represents a number between 0.05 and 1.5, y represents a number from 0 to 2.95 and z = y when M = lithium and zero when M represents a bivalent metal as defined hereinabove, by reacting under solid state conditions a silica source, an alumina source, a magnesia source, a sodium and/or lithium source, a fluoride source and optionally a further source containing A and/or M metal ions and/or a chloride source, at a temperature between the lowest melting point of any alkali fluoride or chloride which is either present as such or which could be formed under solid state synthesis conditions and a temperature not more than 200 °C below said temperature.

The saponites prepared according to the method according to the present invention appear to have a high (hydro)thermal stability which renders them suitable as catalysts or catalyst carriers. It has been found that they can be suitably applied in the isomerisation of straight-chain paraffins such as n-hexane to give products which can be used advantageously in the upgrading of gasoline.

Without wishing to be bound to any particular theory it would appear that the combination of the presence of an alkali fluoride and/or chloride, most likely acting as a kind of flux in the solid state synthesis,

2

EP 0 317 006 B1

and the temperature at which the solid state synthesis is carried out seems to be critical in the preparation of saponites in accordance with the present invention.

The temperature of the solid state mixture should be kept between the (lowest) melting point of an (the) alkali fluoride(s) or chloride(s) present as such or which could be formed under solid state conditions and a value not more than 200 °C below said lowest melting point. Preferably, the process according to the present invention is carried out at a temperature between the (lowest) melting point of an (the) alkali fluoride(s) or chloride(s) present as such or which could be formed under solid state synthesis conditions and a value not more than 100 °C below said melting point. In general, the solid state synthesis in accordance with the present invention will be carried out at a temperature between 600 °C and 1000 °C, provided the criteria given hereinabove are met.

As silica source for the process according to the present invention can suitably be used amorphous silica as well as organic silicon compounds such as silicon esters and silanes which can be converted to silica by methods known in the art or even under certain appropriate process conditions. Normally, the silica source will be subjected to a heat treatment, e.g. calcination up to 800 °C prior to its use in the process according to the present invention.

Suitable alumina sources comprise alumina's such as alpha, gamma or eta-alumina as well as various boehmite forms which can be converted into alumina by methods known in the art or even under certain appropriate synthesis conditions. Also the alumina source is normally subjected to a heat treatment prior to its use in the process according tot the present invention. Also amorphous silica-alumina as well as crystalline (alumino)silicates can be used as silica and/or alumina source in the process according to the present invention. It is of course posible to use a further source of silica and/or alumina in the process according to the present invention.

It should be noted that the silica source and the alumina source should be used normally in such an amount that a molar sum of 4 will be achieved in the general formula II and ascertaining at the same time that the molar ratio $SiO_2/Al_2O_3$ is between 3.33 and 160. Suitably, the molar ratio is below 80. Good results have been obtained using the silica source and the alumina source in such an amount that the molar ratio in the saponite produced is between 4.5 and 40.

Suitable magnesia sources comprise magnesium oxide, magnesium hydroxide as well as magnesium salts of organic acids such as magnesium acetate or propionate. It will be appreciated that magnesium oxide and magnesium acetate are normally subjected to a heat treatment, e.g. at a temperature of up to 800 °C prior to their respective use in the process according to the present invention. The magnesium hydroxide and the magnesium salts of the appropriate organic acids will be normally at least partially converted into magnesia under conditions of the heat treatment. Normally, magnesia or its precursor(s) will be used in such an amount that in the case of the saponites a molar ratio Mg/(Si + Al) of about 0.75 will be achieved, being the stoichiometric ratio for saponites. It should be noted that in case more than one magnesium compound is present in the reaction mixture to be subjected to solid state synthesis to produce the saponites according to the general formula given hereinabove, e.g. when magnesium fluoride is present as (part of) the fluoride source, the molar ratio Mg/(Si + Al) comprises the total molar amount of magnesium compounds present. Preferably,the total molar amount of magnesium oxide or its precursor(s) present in the starting mixture is between 2 and 3 calculated on a Si + Al molar amount of 4. Good results have been obtained using molar amounts ranging from 2.1 to 2.6.

It is mandatory that the solid state synthesis is carried out in the presence of a source of sodium and/or lithium. Suitable sodium compounds comprise sodium carbonate, sodium sulphate, sodium (pyro)phosphate and sodium acetate. Advantageously, use can be made of sodium fluoride since it also serves to introduce a fluoride moiety into the system which is necessary as described hereinafter. Also, sodium chloride can be used, which has the advantage that a chloride source which may optionally be present in the solid state synthesis system is already introduced therein. For lithium compounds similar possibilities exist. Preference is given to the use of sodium fluoride. Normally, the amount of the sodium and/or lithium source to be used in the solid state synthesis starting mixture will be such that the mol fraction of the sodium and/or lithium cation present in tetrahedral positions (expressed as x) in the product satisfies the equation

$$x \geq 4a/(1 + a) \text{ wherein } a = Al/Si. \qquad (III)$$

Normally, an excess of sodium and/or lithium will give very good results.

The solid state synthesis mixture to produce the saponites according to the present invention also requires the presence of a fluoride source, optionally together with a chloride source. It is possible and in fact preferred to use magnesium fluoride as the source of the fluoride required. This has the additional advantage that a magnesium source is introduced at the same time in the solid state synthesis mixture.

<div align="center">3</div>

Another suitable fluoride source comprises sodium fluoride or lithium fluoride, preferably sodium fluoride. It is also possible to use aluminium fluoride as a fluoride source, provided that the proper adjustments with respect to the total amount of alumina (including its precursor(s)) have been taken into account. The amount(s) of fluoride containing component(s) to be used in the starting material is suitably chosen in such a way that allows an (Si + Al)/F molar ratio of about 2 in the saponite produced. It is possible that because of the reaction conditions, and depending to some extent on the amount and nature of the other components present in the solid state synthesis mixture, part of the positions normally occupied by the fluoride moiety will be occupied by a chloride and/or a hydroxyl moiety. It should be noted that normally at least 80% and preferably at least 90% of the positions available for the group Z is indeed occupied by fluorine moieties.

When use is made of silica, alumina, magnesia, sodium fluoride and magnesium fluoride as the constituting ingredients of the solid state synthesis mixture saponites according to the general formula

$$Na_x^+ [(Mg_3)(Si_{4-x}Al_x)O_{10}F_2] \qquad (IV)$$

will be obtained wherein x represents a value as defined hereinbefore. Preferably, the ingredients to be present in the solid state synthesis mixture are chosen such that the mol fraction x in the final product lies in the range between 0.2 and 1.25.

It is also possible to use in addition to the mandatory sources as explained hereinbefore a further source containing A and/or M metal ions and/or a chloride source. A may represent any metal ion having basic or amphoteric properties, including but not limited to sodium, lithium, aluminium and M as defined hereinbefore. It will be appreciated that when one or more compounds containing the moiety A are present under solid state synthesis conditions that moiety or moieties will predominantly be occupying the positions in the structure as reflected by the position of A in the appropriate general formula (II).

In the event that the starting mixture comprises as further metal source an M metal source or sources wherein M represents a bivalent metal ion having an ionic radius between 0.050 and 0.085 nm or lithium, the further metal moiety or moieties are introduced into the octahedral layer at the centre of the triple layer sheet of the saponite obtained. Examples of bivalent metal ions having ionic radii between 0.050 and 0.085 nm comprise iron, nickel, cobalt, manganese, copper and zinc. Mixtures of such bivalent metal ions can also be used. Preference is given to the use of compounds comprising bivalent nickel and/or cobalt.

The amounts of the compound(s) containing M as metal moiety to be used in the solid state synthesis starting material can be chosen within wide limits, provided that the molar fraction of M is below 2.95 and the sum of the molar fractions of the metal M and Mg as present in the saponite-derivative produced equals 3.

When A and M are as defined hereinbefore, the general structure of the saponite-derivative can be represented by the formula

$$A_{(x+z)/n}^{n+} [(Mg_{3-y}M_y)(Si_{4-x}Al_x)O_{10}F_2] \qquad (V)$$

Suitably, the value of y ranges between 0.05 and 2.5, preference being given to values of y between 0.5 and 2.5. It is believed that the compounds according to the general formula V are novel compounds.

It should be noted that when a lithium compound is chosen as representing a source of A, a rather complex structure can be formed since the lithium ion concerned has intrinsically the possibility (as being one of the possible metal ions defined as M) to become present also in the octahedral layer present in the saponite-derivative obtained.

When use is made of nickel as the moiety representing M, the process according to the present process will produce nickel-saponites according to the general formula

$$A_{x/n}^{n+} [(Mg_{3-y}Ni_y)(Si_{4-x}Al_x)O_{10}F_2] \qquad (VI)$$

wherein A, n, x and y have the meanings as defined hereinbefore. Preference is given to nickel-saponites according to the general formula VI wherein A represents a sodium ion, x ranges between O.20 and 1.25 and y ranges between 0.05 and 2.5, in particular between 0.5 and 2.0.

When use is made of cobalt as the moiety M, the process according to the present invention will produce cobalt-saponites, i.e. compounds according to the general formula VI wherein the symbol Ni is replaced by Co. Preference is given to cobalt-saponites wherein A represents a sodium ion, x ranges between 0.20 and 1.25 and y ranges between 0.05 and 2.5, in particular between 0.5 and 2.0.

The process according to the present invention is suitably carried out by mixing the ingredients, if desired after milling to obtain them in a finely divided state, in a reactor vessel of a suitable material such as quartz and subjecting them for a prolonged period of time to a heat treatment allowing the formation of the appropriate saponite. After cooling down the reaction mixture, the product, i.e. the swellable clay can be isolated by making use of its swellability. The reaction mixture is suitably transferred to a vessel containing water which allows the product to swell whilst the insoluble starting materials left over and non-swellable compounds which may have been formed remain in solid form in the system. Preferably, the isolation procedure is carried out under stirring. Stirring may be effected at ambient or elevated temperature, preference being given to temperatures in the range between 20 and 90 °C. The swellable clay (in the form of a suspension in water) is removed and sedimented by treatment with a sedimentation agent such as ammonium chloride or sodium chloride or a similar compound. The clay obtained is suitably separated from the water by decantation or centrifugation. If desired, the clay obtained can be subjected to one or more washing steps to remove traces of salt(s) remaining prior to further drying.

It may be useful to subject the material formed during the solid state synthesis to a treatment with a complexing agent to remove part or all of the components present which substantially can prevent swelling of the swellable materials formed.

It will be appreciated that the swellable clays produced under solid state synthesis conditions may absorb water during one or more stages in the working-up procedure which results in the formation of the corresponding hydrated forms of the synthetic saponites or derivatives thereof prepared in accordance with the present invention. Such (partially) hydrated products are also contemplated to be within the scope of the present invention.

The saponites and saponite-derivatives according to the present invention can be suitably subjected to cross-linking by methods known in the art to obtain so-called pillared clays which are of interest as (catalyst) carriers in hydrocarbon conversion processes and as adsorbents and molecular sieves. Cross-linking can be carried out suitably by using inorganic oligomers or suitable organic or organometallic cations.

The process according to the present invention will now be illustrated by means of the following Examples.

## EXAMPLE 1

A saponite according to the general formula IV was prepared by heating a mixture of predried silica, alumina, sodium fluoride, magnesia and magnesium fluoride (35 grammes in a molar ratio $SiO_2:Al_2O_3:NaF:MgO:MgF_2 = 3.40:0.30:0.75:2.2:1.0$) in a vessel at a temperature of 930 °C for a period of 14 hours. After cooling down, the reaction mixture was transferred to a vessel containing 4 litres of water and kept under stirring at 80 °C for a period of 4 hours. The non-swellable components were allowed to sediment whilst the swellable product fraction was suspended in the water. The suspended clay was removed from the other solid substances present and thereafter treated with a solution of 1 M sodium chloride to allow sedimentation of the clay. The clay thus obtained was collected via centrifugation. Its yield after washing and drying amounted to 13.4 grammes (38.2% on intake). Elemental analysis of the product gave the following composition (%m/m):Al 3.9; Si 23.4; Mg 17.7 and Na 1.0. With reference to the general formula IV the value of x amounted to 0.59. The molar fractions of Si, Al and Mg in the structure amounted to 3.42:0.59:2.99.

## EXAMPLE 2

The process as described in Example 1 was repeated using the same total amount of ingredients in the following molar ratio: $SiO_2:Al_2O_3:NaF:MgO: MgF_2 = 3.20:0.4:1.0:2.2:1.0$. After the synthesis and working-up procedure as described in the previous Example 23.5 grammes of swellable saponite could be obtained (67.4 % on intake). Elemental analysis of the product gave the following composition (%m/m):Al 5.4; Si 21.6; Mg 17.2 and Na 1.94. The value of x amounted to 0.83. The molar fractions of Si, Al and Mg in the structure amounted to 3.21:0.84:2.95.

## EXAMPLE 3

The process as described in Example 1 was repeated using a solid state synthesis temperature of 940 °C and a molar ratio of the ingredients amounting to: $SiO_2:Al_2O_3:NaF:MgO: MgF_2 = 3.10:0.45:1.125:2.2:1.0$. After the synthesis and working-up procedure as described in the previous Example 20.1 grammes of swellable saponite could be obtained (57.4% on intake). Elemental analysis of the product gave the following composition C %m/m): Al 5.8; Si 21.5; Mg 18.0 and Na 2.59. The value of x amounted to 0.87. The molar fractions of Si, Al and Mg in the structure amounted to 3.11:0.87:3.02.

## EXAMPLE 4

The process as described in Example 3 was repeated using the ingredients in a molar ratio $SiO_2:Al_2O_3:NaF:MgO:MgF_2 = 2.80:0.60:1.50:2.2:1.0$. After the solid state synthesis and working-up procedure as described in the previous Example 14.0 grammes of swellable saponite could be obtained (39.4% on intake). Elemental analysis of the product gave the following composition (%m/m): Al 7.7; Si 19.4; Mg 18.6 and Na 1.7. The value for x amounted to 1.17. The molar fractions of Si, Al and Mg in the structure amounted to 2.78:1.14:3.08.

## EXAMPLE 5

The experiment as described in Example 1 was repeated at a temperature of 920 °C and using the ingredients in a molar ratio: $SiO_2:Al_2O_3:NaF:MgO:MgF_2 = 3.80:0.1:0.5:2.2:1.0$. After cooling down and treatment with a solution of ammonium chloride 12.7 grammes of swellable saponite were obtained (36.3% on intake). Elemental analysis of the product gave the following composition (%m/m): Al 1.8; Si 26.6; Mg 18.0 and Na 1.2.

## EXAMPLE 6

The experiment as described in Example 1 was repeated at a temperature of 850 °C using sodium pyrophosphate as the sodium source. The ingredients were used in a molar ratio: $SiO_2:Al_2O_3:Na_4P_2O_7:MgO:MgF_2 = 3.60:0.20:0.125:2.2:1.0$. After the synthesis and the working-up procedure as described in Example 1 13.5 grammes of a swellable saponite were obtained (35.5% on intake).

## EXAMPLE 7

An experiment as described in Example 1 was carried out at a temperature of 920 °C and using in addition aluminium fluoride as a further source of both fluoride and aluminium. The ingredients were used in a molar ratio: $SiO_2:Al_2O_3:AlF_3:NaF:MgO:MgF_2 = 3.70:0.10:0.10:0.5:2.2.:1.0$. After cooling down and a working-up procedure using ammonium chloride 15.2 grammes of swellable saponite were obtained (43.4% on intake).

## EXAMPLE 8

An experiment as described in Example 1 was carried out at a temperature of 790 °C and using sodium chloride in stead of sodium fluoride. The ingredients were used in a molar ratio: $SiO_2:Al_2O_3:NaCl:MgO:MgF_2 = 3.67:0.167:1.0:2.2:3.0$. After cooling down and a working-up procedure using sodium chloride 17.7 grammes of swellable saponite were obtained (50.6% on intake).

## EXAMPLE 9

A nickel-saponite according to the general formula VI was prepared by heating 35 grammes of a mixture of predried ingredients having a molar ratio: $SiO_2:Al_2O_3:NaF:MgO:MgF_2:NiO:NiF_2 = 3.67:0.167:1.0:1.5:0.75:0.75:0.75$. After cooling down and treatment with a solution of ammonium chloride 18.9 grammes of swellable nickel-saponite could be obtained (53.9% on intake). Elemental analysis of the product gave the following composition (%m/m): Al 2.6; Si 22.9; Mg 10.2; Ni 15.2 and Na 0.76. The value of x as described in formula VI amounted to 0.42. The molar fractions of Si, Al, Mg and Ni amounted to 3.59:0.42:1.85:1.14.

EXAMPLE 10

The experiment as described in the previous Example was repeated using the ingredients in the molar ratio: $SiO_2:Al_2O_3:NaF:MgO:MgF_2:NiO:NiF_2 = 3.67:0.167:1.0:0.75:0.375:1.5:1.5$. After cooling down and treatment with ammonium chloride 14 grammes of swellable nickel-saponite could be obtained (40% on intake). Elemental analysis of the product gave (%m/m): Al 3.14; Si 19.7; Mg 5.1; Ni 25.8 and Na 1.46. The value of x amounted to 0.57. The molar fractions of Si, Al, Mg and Ni amounted to 3.35:0.56:1.00:2.10.

EXAMPLE 11

A cobalt-saponite according to the general formula VI (wherein the symbol Ni is replaced by the symbol Co) was prepared by heating at 920 °C 35 grammes of a mixture having the molar ratio: $SiO_2:Al_2O_3:NaF:MgO:MgF_2:CoO = 3.67:0.17:1.0:1.5:0.75:0.75$. After cooling down and treatment with ammonium chloride 15.9 grammes of swellable cobalt-saponite could be obtained (45.5% on intake). Elemental analysis of the product gave (%m/m): Al 2.6; Si 23.5; Mg 12.8; Co 8.6 and Na 1.8. The value of x amounted to 0.41. The molar fractions of Si, Al, Mg and Co amounted to 3.65:0.42:2.29:0.65.

EXAMPLE 12

A copper-saponite according to the general formula VI (wherein the symbol Ni is replaced by the symbol Cu) was prepared by heating at 920 °C 35 grammes of a mixture having the molar ratio: $SiO_2:Al_2O_3:NaF:MgO:MgF_2:CuO = 3.50:0.25:1.0:1.4:0.7:1.2$. After cooling down and treatment with ammonium chloride swellable copper-saponite could be obtained. Elemental analysis of the product gave (% m/m): Al 4.3; Si 21.9; Mg 13.2; Cu 5.2 and Na 4.37. The value of x amounted to 0.68. The molar fractions of Si, Al, Mg and Cu amounted to 3.49:0.72:2.43:0.37.

EXAMPLE 13

The catalytic activity of the saponites according to the present invention was tested by measuring the hydro-isomerisation activity towards n-hexane at a temperature of 340 °C. This was done by converting the appropriate saponite (derivative) into its protonic form by calcination and testing it in the presence of platinum at a WHSV of 2 and a hydrogen/n-hexane molar ratio of 30. When the saponite as described in Example 5 was subjected to the n-hexane isomerisation test a conversion of 6.6% was obtained and a selectivity towards $C_6$-isomers of 81.8 % and towards $nC_5$ of 5.4%. When the nickel-saponite as described in Example 10 was subjected to the same isomerisation test its conversion amounted to 23% and its selectivity towards $C_6$-isomers was 2.6% and towards $nC_5$ 56.0%.

**Claims**
**Claims for the following Contracting States : BE, CH, DE, FR, GB, IT, LI, NL, SE**

1. Process for the manufacture of synthetic saponites according to the general formula:

$$A^{n+}_{(x+z)/n}[(Mg_{3-y}M_y)\ (Si_{4-x}Al_x)O_{10}Z_2] \qquad (II)$$

wherein A represents any metal ion having basic or amphoteric properties, M represents a bivalent metal ion having an ionic radius between 0.050 nm and 0.085 nm or lithium, Z represents a fluoride moiety, n represents the valency of A, x represents a number between 0.05 and 1.5, y represents a number from 0 to 2.95, and z = y when M = lithium and zero when M represents a bivalent metal ion as defined hereinabove, by reacting under solid state conditions a silica source, an alumina source, a magnesia source, a sodium and/or lithium source, a fluoride source and optionally a further source containing A and/or M metal ions and/or a chloride source, at a temperature between the lowest melting point of any alkali fluoride or chloride which is either present as such or could be formed under solid state conditions and a temperature not more than 200 °C below said temperature.

2. Process according to claim 1, wherein the solid state synthesis is carried out at a temperature between the lowest melting point of any alkali fluoride or chloride which is either present as such or which could be formed under the reaction conditions and a temperature not more than 100 °C below said temperature.

3. Process according to claim 1 or 2, wherein use is made of silica and alumina in a molar ratio between 3.33 and 160.

4. Process according to claim 3, wherein use is made of silica and alumina in a molar ratio below 80, preferably between 4.5 and 40.

5. Process according to one or more of claims 1-4, wherein use is made of magnesia (or a precursor thereof) in the starting mixture in a molar amount between 2 and 3 calculated on a Si + Al molar amount of 4, preferably in a molar amount ranging from 2.1 to 2.6 calculated on a Si + Al molar amount of 4.

6. Process according to one or more of claims 1-5, wherein use is made of sodium carbonate, sodium sulphate, sodium (pyro)phosphate, sodium chloride, sodium fluoride or sodium acetate as the sodium source(s).

7. Process according to one or more of claims 1-6, wherein use is made of magnesium fluoride as the source of fluoride.

8. Process according to one or more of claims 1-7, wherein use is made of a further metal source M wherein M represents a bivalent metal ion having an ionic radius between 0.050 and 0.085 nm, preferably nickel and/or cobalt, or lithium.

9. Process according to claim 8, wherein use is made of an amount of a further metal source M allowing a molar fraction of M below 2.95 whilst the sum of the molar fractions of M and Mg equals 3 in the saponite according to formula II.

10. Saponite-derivatives according to the general formula

$$A^{n+}_{(x+z)/n}[(Mg_{3-y}M_y)(Si_{4-x}Al_x)O_{10}F_2] \qquad (V)$$

wherein A represents any metal ion having basic or amphoteric properties, M represents a bivalent metal ion having an ionic radius between 0.050 and 0.0850 nm or lithium, x represents a number between 0.05 and 1.5, y represents a number between 0.05 and 2.5 and z = y when M = lithium and zero when M represents a bivalent metal ion having an ionic radius between 0.050 nm and 0.0850 nm.

11. Saponite-derivatives according to claim 10, wherein y represents a number between 0.5 and 2.0.

12. Saponite-derivatives according to claim 10 or 11, wherein M represents a nickel or cobalt moiety.

13. Saponite-derivatives according to claim 12, wherein the saponite-derivatives contain sodium as the moiety A, x ranges between 0.20 and 1.25 and y ranges between 0.05 and 2.5.

14. Use of saponites as prepared according to one or more of claims 1-9 and/or saponite-derivatives as described in one or more of claims 10-13 as (catalyst) carriers.

15. Process for the (hydro)conversion, in particular (hydro)isomerization of hydrocarbons wherein one or more saponites as prepared according to one or more of claims 1-9 and/or saponite-derivatives as described in one or more of claims 10-13 are used as (catalyst) carriers.

**Claims for the following Contracting State : ES**

1. Process for the manufacture of synthetic saponites according to the general formula:

$$A^{n+}_{(x+z)/n}[(Mg_{3-y}M_y)\ (Si_{4-x}Al_x)O_{10}Z_2] \qquad (II)$$

wherein A represents any metal ion having basic or amphoteric properties, M represents a bivalent metal ion having an ionic radius between 0.050 nm and 0.085 nm or lithium, Z represents a fluoride moiety, n represents the valency of A, x represents a number between 0.05 and 1.5, y represents a number from 0 to 2.95, and z = y when M = lithium and zero when M represents a bivalent metal ion as defined hereinabove, by reacting under solid state conditions a silica source, an alumina source, a magnesia source, a sodium and/or lithium source, a fluoride source and optionally a further source containing A and/or M metal ions and/or a chloride source, at a temperature between the lowest melting point of any alkali fluoride or chloride which is either present as such or could be formed under solid state conditions and a temperature not more than 200 °C below said temperature.

2. Process according to claim 1, wherein the solid state synthesis is carried out at a temperature between the lowest melting point of any alkali fluoride or chloride which is either present as such or which could be formed under the reaction conditions and a temperature not more than 100 °C below said temperature.

3. Process according to claim 1 or 2, wherein use is made of silica and alumina in a molar ratio between 3.33 and 160.

4. Process according to claim 3, wherein use is made of silica and alumina in a molar ratio below 80, preferably between 4.5 and 40.

5. Process according to one or more of claims 1-4, wherein use is made of magnesia (or a precursor thereof) in the starting mixture in a molar amount between 2 and 3 calculated on a Si + Al molar amount of 4, preferably in a molar amount ranging from 2.1 to 2.6 calculated on a Si + Al molar amount of 4.

6. Process according to one or more of claims 1-5, wherein use is made of sodium carbonate, sodium sulphate, sodium (pyro)phosphate, sodium chloride, sodium fluoride or sodium acetate as the sodium source(s).

7. Process according to one or more of claims 1-6, wherein use is made of magnesium fluoride as the source of fluoride.

8. Process according to one or more of claims 1-7, wherein use is made of a further metal source M wherein M represents a bivalent metal ion having an ionic radius between 0.050 and 0.085 nm, preferably nickel and/or cobalt or lithium.

9. Process according to claim 8, wherein use is made of an amount of a further metal source M allowing a molar fraction of M below 2.95 whilst the sum of the molar fractions of M and Mg equals 3 in the saponite according to formula II.

10. Use of saponites as prepared according to one or more of claims 1-9 as (catalyst) carriers.

11. Process for the (hydro)conversion, in particular (hydro)isomerization of hydrocarbons wherein one or more saponites as prepared according to one or more of claims 1-9 are used as (catalyst) carriers.

**Patentansprüche**
**Patentansprüche für folgende Vertragsstaaten : BE, CH, DE, FR, GB, IT, LI, NL, SE**

1.  Verfahren zur Herstellung von synthetischen Saponiten der allgemeinen Formel:

$$A^{n+}_{(x+z)n}[(Mg_{3-y}M_y)(Si_{4-x}Al_x)O_{10}Z_2] \qquad (II)$$

in der A irgendein Metallion mit basischen oder amphoteren Eigenschaften ist, M ein zweiwertiges Metallion mit einen Ionenradius zwischen 0,050 nm und 0,085 nm oder Lithium ist, Z einen Fluoridrest darstellt, n die Wertigkeit von A bedeutet, x eine Zahl zwischen 0,05 und 1,5 ist, y eine Zahl von 0 bis 2,95 ist und z = y, wenn M Lithium ist, und z = 0, wenn M ein zweiwertiges Metallion, wie oben definiert, darstellt, durch Umsetzen unter Festphasenbedingungen einer Siliciumdioxidquelle, einer Aluminiumoxidquelle, einer Magnesiumoxidquelle, einer Natrium- und/oder Lithiumquelle, einer Fluoridquelle und gegebenenfalls einer weiteren Quelle, enthaltend A- und/oder M-Metallionen, und/oder einer Chloridquelle, bei einer Temperatur zwischen dem niedrigsten Schmelzpunkt eines Alkalifluorids oder -chlorids, das entweder als solches vorliegt oder sich unter Festphasenbedingungen bilden könnte, und einer Temperatur von nicht mehr als 200°C unterhalb der genannten Schmelztemperatur.

2.  Verfahren nach Anspruch 1, in dem die Festphasensynthese bei einer Temperatur zwischen dem niedrigsten Schmelzpunkt irgendeines Alkalifluorids oder -chlorids, das entweder als solches vorliegt oder sich unter den Reaktionsbedingungen bilden könnte, und einer Temperatur von nicht mehr als 100°C unterhalb der genannten Temperatur durchgeführt wird.

3.  Verfahren nach Anspruch 1 oder 2, in dem Siliciumdioxid und Aluminiumoxid in einem Molverhältnis von 3,33 bis 160 eingesetzt werden.

4.  Verfahren nach Anspruch 3, in dem Siliciumdioxid und Aluminiumoxid in einem Molverhältnis unter 80, vorzugsweise zwischen 4,5 und 40, eingesetzt werden.

5.  Verfahren nach einem oder mehreren der Ansprüche 1 bis 4, in dem Magnesiumoxid (oder ein Vorläufer davon) in der Ausgangsmischung in einer molaren Menge zwischen 2 und 3, bezogen auf eine Molmenge von Si + Al von 4, vorzugsweise in einer molaren Menge im Bereich von 2,1 bis 2,6, bezogen auf die molare Menge von Si + Al von 4, eingesetzt wird.

6.  Verfahren nach einem oder mehreren der Ansprüche 1 bis 5, in dem Natriumcarbonat, Natriumsulfat, Natrium(pyro)phosphat, Natriumchlorid, Natriumfluorid oder Natriumacetat als Natriumquelle(n) eingesetzt wird (werden).

7.  Verfahren nach einem oder mehreren der Ansprüche 1 bis 6, in dem Magnesiumfluorid als Fluoridquelle eingesetzt wird.

8.  Verfahren nach einem oder mehreren der Ansprüche 1 bis 7, in dem eine weitere Metallquelle M, wobei M ein zweiwertiges Metallion mit einem Ionenradius zwischen 0,050 nm und 0,085 nm, vorzugsweise Nickel und/oder Kobalt oder Lithium ist, eingesetzt wird.

9.  Verfahren nach Anspruch 8, in dem eine Menge einer weiteren Metallquelle M, die eine Molfraktion von M unterhalb 2,95 ermöglicht, während die Summe der Molfraktionen von M und Mg im Saponit der Formel II 3 ist, eingesetzt wird.

10. Saponit-Derivate nach der allgemeinen Formel

$$A^{n+}_{(x+z)/n}[(Mg_{3-y}M_y)(Si_{4-x}Al_x)O_{10}F_2] \qquad (V)$$

in der A irgendein Metallion mit basischen oder amphoteren Eigenschaften ist, M ein zweiwertiges Metallion mit einem Ionenradius zwischen 0,050 nm und 0,0850 nm oder Lithium ist, x eine Zahl zwischen 0,05 und 1,5 ist, y eine Zahl zwischen 0,05 und 2,5 ist und $z = y$, wenn M = Lithium, und $z = 0$, wenn M ein zweiwertiges Metallion mit einem Ionenradius zwischen 0,050 nm und 0,0850 nm ist.

11. Saponit-Derivate nach Anspruch 10, in denen y eine Zahl zwischen 0,5 und 2,0 ist.

12. Saponit-Derivate nach Anspruch 10 oder 11, in denen M einen Nickel- oder Kobaltrest bedeutet.

13. Saponit-Derivate nach Anspruch 12, wobei die Saponit-Derivate Natrium als Rest A enthalten, x im Bereich zwischen 0,20 und 1,25 liegt und y im Bereich zwischen 0,05 und 2,5 liegt.

14. Verwendung von Saponiten, die nach einem oder mehreren der Ansprüche 1 bis 9 hergestellt worden sind, und/oder von Saponit-Derivaten, wie in einem oder mehreren der Ansprüche 10 bis 13 beschrieben, als (Katalysator)träger.

15. Verfahren zur (Hydro)umwandlung, vor allem zur (Hydro)isomerisierung von Kohlenwasserstoffen, in dem ein oder mehrere Saponit(e), hergestellt nach einem oder mehreren der Ansprüche 1 bis 9, und/oder Saponit-Derivate, wie in einem oder mehreren der Ansprüche 10 bis 13 beschrieben, als (Katalysator)träger eingesetzt werden.

**Patentansprüche für folgenden Vertragsstaat : ES**

1. Verfahren zur Herstellung von synthetischen Saponiten der allgemeinen Formel:

$$A_{(x+z)n}^{n+}[(Mg_{3-y}M_y)(Si_{4-x}Al_x)O_{10}Z_2] \qquad (II)$$

in der A irgendein Metallion mit basischen oder amphoteren Eigenschaften ist, M ein zweiwertiges Metallion mit einen Ionenradius zwischen 0,050 nm und 0,085 nm oder Lithium ist, Z einen Fluoridrest darstellt, n die Wertigkeit von A bedeutet, x eine Zahl zwischen 0,05 und 1,5 ist, y eine Zahl von 0 bis 2,95 ist und $z = y$, wenn M Lithium ist, und $z = 0$, wenn M ein zweiwertiges Metallion, wie oben definiert, darstellt, durch Umsetzen unter Festphasenbedingungen einer Siliciumdioxidquelle, einer Aluminiumoxidquelle, einer Magnesiumoxidquelle, einer Natrium- und/oder Lithiumquelle, einer Fluoridquelle und gegebenenfalls einer weiteren Quelle, enthaltend A- und/oder M-Metallionen, und/oder einer Chloridquelle, bei einer Temperatur zwischen dem niedrigsten Schmelzpunkt eines Alkalifluorids oder -chlorids, das entweder als solches vorliegt oder sich unter Festphasenbedingungen bilden könnte, und einer Temperatur von nicht mehr als 200 °C unterhalb der genannten Schmelztemperatur.

2. Verfahren nach Anspruch 1, in dem die Festphasensynthese bei einer Temperatur zwischen dem niedrigsten Schmelzpunkt irgendeines Alkalifluorids oder -chlorids, das entweder als solches vorliegt oder sich unter den Reaktionsbedingungen bilden könnte, und einer Temperatur von nicht mehr als 100 °C unterhalb der genannten Temperatur durchgeführt wird.

3. Verfahren nach Anspruch 1 oder 2, in dem Siliciumdioxid und Aluminiumoxid in einem Molverhältnis von 3,33 bis 160 eingesetzt werden.

4. Verfahren nach Anspruch 3, in dem Siliciumdioxid und Aluminiumoxid in einem Molverhältnis unter 80, vorzugsweise zwischen 4,5 und 40, eingesetzt werden.

5. Verfahren nach einem oder mehreren der Ansprüche 1 bis 4, in dem Magnesiumoxid (oder ein Vorläufer davon) in der Ausgangsmischung in einer molaren Menge zwischen 2 und 3, bezogen auf eine Molmenge von Si + Al von 4, vorzugsweise in einer molaren Menge im Bereich von 2,1 bis 2,6, bezogen auf die molare Menge von Si + Al von 4, eingesetzt wird.

6. Verfahren nach einem oder mehreren der Ansprüche 1 bis 5, in dem Natriumcarbonat, Natriumsulfat, Natrium(pyro)phosphat, Natriumchlorid, Natriumfluorid oder Natriumacetat als Natriumquelle(n) eingesetzt wird (werden).

**7.** Verfahren nach einem oder mehreren der Ansprüche 1 bis 6, in dem Magnesiumfluorid als Fluoridquelle eingesetzt wird.

**8.** Verfahren nach einem oder mehreren der Ansprüche 1 bis 7, in dem eine weitere Metallquelle M, wobei M ein zweiwertiges Metallion mit einen Ionenradius zwischen 0,050 nm und 0,085 nm, vorzugsweise Nickel und/oder Kobalt oder Lithium ist, eingesetzt wird.

**9.** Verfahren nach Anspruch 8, in dem eine Menge einer weiteren Metallquelle M, die eine Molfraktion von M unterhalb 2,95 ermöglicht, während die Summe der Molfraktionen von M und Mg im Saponit der Formel II 3 ist, eingesetzt wird.

**10.** Verwendung von Saponiten, die nach einem oder mehreren der Ansprüche 1 bis 9 hergestellt worden sind, als (Katalysator)träger.

**11.** Verfahren zur (Hydro)umwandlung, vor allem zur (Hydro)isomerisierung von Kohlenwasserstoffen, in dem ein oder mehrere Saponit(e), hergestellt nach einem oder mehreren der Ansprüche 1 bis 9, als (Katalysator)träger eingesetzt werden.

**Revendications**

**Revendications pour les Etats contractants suivants : BE, CH, DE, FR, GB, IT, LI, NL, SE**

**1.** Procédé de préparation de saponites synthétiques selon la formule :

$$A^{n+}_{(x+z)/n}[(Mg_{3-y}M_y)\ (Si_{4-x}Al_x)O_{10}Z_2] \qquad (II)$$

où A représente tout ion métallique ayant des propriétés basiques ou amphotériques, M représente un ion métallique bivalent ayant un rayon ionique compris entre 0,050 nm et 0,085 nm ou le lithium, Z représente une partie fluorure, n représente la valence de A, x représente un nombre compris entre 0,05 et 1,5, y représente un nombre allant de 0 à 2,95 et z = y lorsque M = lithium et zéro lorsque M représente un métal bivalent tel que défini ci-dessus, en faisant réagir dans des conditions d'état solide une source de silice, une source d'aluminium, une source de magnésie, une source de sodium et/ou de lithium, une source de fluorure et facultativement une source contenant les ions métalliques A et/ou M et/ou une source de chlorure, à une température comprise entre le plus bas point de fusion de tout fluorure ou chlorure alcalin qui est présent tel quel ou peut être formé dans des conditions de synthèse à l'état solide et à une température ne dépassant pas 200°C en dessous de ladite température.

**2.** Procédé selon la revendication 1, dans lequel la synthèse à l'état solide est conduite à une température comprise entre le plus faible point de fusion de tout fluorure ou chlorure alcalin qui est présent tel quel ou qui peut être formé dans les conditions de la réaction et une température ne dépassant pas 100°C en dessous de ladite température.

**3.** Procédé selon la revendication 1 ou 2, dans lequel on utilise de la silice et de l'alumine dans un rapport molaire compris entre 3,33 et 160.

**4.** Procédé selon la revendication 3, dans lequel on utilise de la silice et de l'alumine dans un rapport molaire inférieur à 80, de préférence compris entre 4,5 et 40.

**5.** Procédé selon une ou plusieurs des revendications 1-4, où l'on utilise de la magnésie (dans le mélange de départ en une quantité molaire comprise entre 2 et 3 calculée sur un rapport molaire Si + Al de 4, de préférence en une quantité molaire allant de 2,1 à 2,6, calculée sur une quantité molaire de si + Al de 4.

**6.** Procédé selon une ou plusieurs des revendications 1-5, dans lequel on utilise le carbonate de sodium, le sulfate de sodium, le (pyro)phosphate de sodium, le chlorure de sodium, le fluorure de sodium ou l'acétate de sodium comme source(s) de sodium.

**7.** Procédé selon une ou plusieurs des revendications 1-6, dans lequel on utilise le fluorure de magnésium comme source de fluorure.

**8.** Procédé selon une ou plusieurs des revendications 1-7, dans lequel on utilise une autre source métallique M, dans laquelle M représente un ion métallique bivalent ayant un rayon ionique compris entre 0,050 et 0,085 nm, de préférence le nickel etLou le cobalt ou le lithium.

**9.** Procédé selon la revendication 8, dans lequel on utilise une quantité d'une source de métal M permettant d'obtenir une fraction molaire de M inférieure à 2,95 tandis que la somme des fractions molaires de M et Mg est égale à 3 dans la saponite selon la formule II.

**10.** Dérivés de saponite selon la formule générale

$$A^{n+}_{(x+z)/n}[(Mg_{3-y}M_y)(Si_{4-x}Al_x)O_{10}F_2] \qquad (V)$$

dans laquelle A représente tout ion métallique ayant des propriétés basiques ou amphotères, M représente un ion métallique bivalent ayant un rayon ionique compris entre 0,050 et 0,850 nm ou le lithium, x représente un nombre compris entre 0,05 et 1,5, y représente un nombre compris entre 0,05 et 2,5 et z = y lorsque M = lithium et zéro lorsque M représente un ion métallique bivalent ayant un rayon ionique compris entre 0,050 nm et 0,0850 nm.

**11.** Dérivés de saponite selon la revendication 10, où y représente un nombre compris entre 0,5 et 2,0.

**12.** Dérivés de saponite selon la revendication 10 ou 11, où M représente une fraction nickel ou cobalt.

**13.** Dérivés de saponite selon la revendication 12, où les dérivés de saponite contiennent du sodium comme fraction A, x se situe entre 0,20 et 1,25, et y se situe entre 0,05 et 2,5.

**14.** Utilisation de saponites telles que préparées selon une ou plusieurs des revendications 1-9 et/ou de dérivés de saponite tels que décrits dans une ou plusieurs des revendications 10-13 comme supports (catalyseurs).

**15.** Procédé d'(hydro)transformation, en particulier d'(hydro)isomérisation d'hydrocarbures où l'on prépare une ou plusieurs saponites selon une ou plusieurs des revendications 1-19 et/ou l'on utilise des dérivés de saponite tels que décrits dans une ou plusieurs des revendications 10-13 comme supports (catalyseurs).

**Revendications pour l'Etat contractant suivant : ES**

**1.** Procédé de préparation de saponites synthétiques selon la formule :

$$A^{n+}_{(x+z)/n}[(Mg_{3-y}M_y)(Si_{4-x}Al_x)O_{10}Z_2] \qquad (II)$$

où A représente tout ion métallique ayant des propriétés basiques ou amphotériques, M représente un ion métallique bivalent ayant un rayon ionique compris entre 0,050 nm et 0,085 nm ou le lithium, Z représente une partie fluorure, n représente la valence de A, x représente un nombre compris entre 0,05 et 1,5, y représente un nombre allant de 0 à 2,95 et z = y lorsque M = lithium et zéro lorsque M représente un métal bivalent tel que défini ci-dessus, en faisant réagir dans des conditions d'état solide une source de silice, une source d'aluminium, une source de magnésie, une source de sodium et/ou de lithium, une source de fluorure et facultativement une source contenant les ions métalliques A et/ou M et/ou une source de chlorure, à une température comprise entre le plus bas point de fusion de tout fluorure ou chlorure alcalin qui est présent tel quel ou peut être formé dans des conditions de synthèse à l'état solide et à une température ne dépassant pas 200 ° C en dessous de ladite température.

2. Procédé selon la revendication 1, dans lequel la synthèse à l'état solide est conduite à une température comprise entre le plus faible point de fusion de tout fluorure ou chlorure alcalin qui est présent tel quel ou qui peut être formé dans les conditions de la réaction et une température ne dépassant pas 100°C en dessous de ladite température.

3. Procédé selon la revendication 1 ou 2, dans lequel on utilise de la silice et de l'alumine dans un rapport molaire compris entre 3,33 et 160.

4. Procédé selon la revendication 3, dans lequel on utilise de la silice et de l'alumine dans un rapport molaire inférieur à 80, de préférence compris entre 4,5 et 40.

5. Procédé selon une ou plusieurs des revendications 1-4, où l'on utilise de la magnésie (dans le mélange de départ en une quantité molaire comprise entre 2 et 3 calculée sur un rapport molaire Si + Al de 4, de préférence en une quantité molaire allant de 2,1 à 2,6, calculée sur une quantité molaire de si + Al de 4.

6. Procédé selon une ou plusieurs des revendications 1-5, dans lequel on utilise le carbonate de sodium, le sulfate de sodium, le (pyro)phosphate de sodium, le chlorure de sodium, le fluorure de sodium ou l'acétate de sodium comme source(s) de sodium.

7. Procédé selon une ou plusieurs des revendications 1-6, dans lequel on utilise le fluorure de magnésium comme source de fluorure.

8. Procédé selon une ou plusieurs des revendications 1-7, dans lequel on utilise une autre source métallique M, dans laquelle M représente un ion métallique bivalent ayant un rayon ionique compris entre 0,050 et 0,085 nm, de préférence le nickel etLou le cobalt ou le lithium.

9. Procédé selon la revendication 8, dans lequel on utilise une quantité d'une source de métal M permettant d'obtenir une fraction molaire de M inférieure à 2,95 tandis que la somme des fractions molaires de M et Mg est égale à 3 dans la saponite selon la formule II.

10. Utilisation de saponites telles que préparées selon une ou plusieurs des revendications 1-9 comme supports (catalyseurs).

11. Procédé d'(hydro)transformation, en particulier d'(hydro)isomérisation d'hydrocarbures dans lequel on utilise comme supports (catalyseurs) une ou plusieurs saponites telles que préparées selon une ou plusieurs des revendications 1-9.